# EUROPÄISCHE PATENTSCHRIFT

(11) **EP 1 590 366 B1**
(45) Veröffentlichungstag und Bekanntmachung des Hinweises auf die Patenterteilung: **07.05.2008**
(21) Anmeldenummer: 04701939.3
(22) Anmeldetag: 14.01.2004
(51) Int. Cl.: C07K 7/06, A61K 38/08

(54) **PEPTIDISCHE SULFONAMIDE**
PEPTIDIC SULFONAMIDES
SULFONAMIDES PEPTIDIQUES

(30) Priorität: 06.02.2003 EP 03002556
(43) Veröffentlichungstag der Anmeldung: 02.11.2005
(73) Patentinhaber: Merck Patent GmbH, 64293 Darmstadt (DE)
(72) Erfinder: JONCZYK, Alfred, 64295 Darmstadt (DE); GROTH, Ulrich, 78464 Konstanz (DE); ZISCHINSKY, Gunther, 10783 Berlin (DE)
(86) Internationale Anmeldenummer: PCT/EP2004/000211
(87) Internationale Veröffentlichungsnummer: WO 2004/069861

(56) Entgegenhaltungen:
- WO-A-00/37487
- DE-A- 19 929 410
- US-B1- 6 245 809
- THE JOURNAL OF BILOGICAL CHEMISTRY, Bd. 277, Nr. 37, 13. September 2002 (2002-09-13), Seiten 33564-33570, XP002287086

### SEQUENCE LISTING

<110> Merck Patent GmbH
<120> Peptidische Sulfonamide
<130> P03/012-DO
<140> PCT/EP2004/000211
   <141> 2004-01-14
<160> 1
<170> PatentIn version 3.1
<210> 1
   <211> 8
   <212> PRT
   <213> Artificial
<220>
   <223> All peptides are acetylated (Ac-) on the N-terminus and amidated (-NH2) on the C-terminus (or carboxy-terminus)
<220>
   <221> MISC_FEATURE
   <222> (2)..(2)
   <223> Xaa at position 2 is Dap(Psa) but Dap(F5-Psa), Dap(2-NO2-Psa), Dap(4-NO2-Psa), Dap(2,4-NO2-Psa), Dap(6-OMe-Psa), Dap(2-CF3-Psa), Dap(3-CF3-Psa), Dap(Me5-Psa), Dap(4-tBu-Psa), Dap(Bsa), Dap(iPrs), Dap(1-Nap), Dap(2-Nap) and Dap(4-Ph-Psa) are also considered.
<400> 1
   Arg Xaa Asp Leu Asp Ser Leu Arg
   1               5

## Beschreibung

Die Erfindung betrifft Peptide, der Formel I

R¹-Arg-X-Asp-Leu-Asp-Ser-Leu-Arg-R² I

worin
- R¹: H, Acetyl oder Acyl und
- R²: -OH, OR³ NH₂,NHR³, N(R³)₂
- R³: Alkyl, Aralkyl, Aryl, Het und
- X: eine Aminosäure der Formel II
bedeutet, 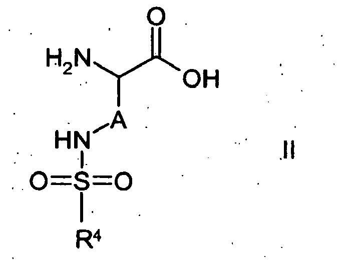 worin
- A: (CH₂)ₙ
- R⁴: Aralkyl oder Aryl, und
- n: 1, 2, 3, 4, 5 oder 6
bedeutet,
und die Aminosäure der Formel II über eine Peptidbindung der α-Aminogruppe mit dem benachbarten Arg und über eine Peptidbindung der α-Carboxygruppe mit der α-Aminogruppe des benachbarten Asp verbunden ist.

Gegenstand der vorliegenden Erfindung sind auch die pharmazeutisch verwendbaren
a) Prodrugs, ausgewählt unter mit Alkyl- oder Acylgruppen, Zuckern oder Oligopeptiden abgewandelten Verbindungen der Formel I,
b) Derivate, ausgewählt unter den N-Methyl-, N-Ethyl-, N-Propyl-, N-Benzyl- und C_{α}-Methylderivaten sowie unter Derivaten, die N-terminal eine Acetylgruppe tragen oder in denen die Acetylgruppe durch eine andere Acylfunktion ersetzt ist, die ausgewählt ist unter Propionyl, Butyryl und Benzoyl,
c) Solvate,
d) Stereoisomere und
e) Salze
   davon, wobei deren Mischungen in allen Verhältnissen umfasst sind.

Der Erfindung lag die Aufgabe zugrunde, Verbindungen mit wertvollen Eigenschaften aufzufinden, insbesondere solche, die zur Herstellung von Arzneimitteln verwendet werden können.

Es wurde gefunden, daß die erfindungsgemäßen Verbindungen der Formel I und ihre Salze bei guter Verträglichkeit sehr wertvolle pharmakologische Eigenschaften besitzen.

Die erfindungsgemäßen Verbindungen der Formel I sind Liganden des Blutplättchenintegrins GPIIbIIIa und den αᵥ-Integrinen, bevorzugt dem αᵥβ₆ und αᵥβ₃ Integrin, und finden somit Verwendung als Agonisten und/oder Antagonisten zur Behandlung verschiedener Krankheiten und pathologischer Befunde. Sie finden weiterhin als Diagnostika oder Reagenzien Anwendung.

Andere Inhibitoren des Integrins αᵥβ₆ sind in der DE 19858857, der DE 19929410, der WO 00/37487 und von S.Kraft et al. in J. Biol. Chem. 274, 1979-85 (1999) beschrieben.

Integrine gehören zu der Familie von heterodimeren Klasse I - Transmembran-Rezeptoren, die in zahlreichen Zell-Matrix- bzw. Zell-Zell-Adhäsionsvorgängen eine wichtige Rolle spielen (Tuckwell et al., 1996, Symp. Soc. Exp. Biol. 47). Sie können grob in drei Klassen eingeteilt werden: die β₁-Integrine, die Rezeptoren für die extrazelluläre Matrix darstellen, die β₂-Integrine, welche auf Leukozyten aktivierbar sind und während inflammatorischen Prozessen "getriggert" werden, sowie die αᵥ-Integrine, die die Zellantwort bei Wundheilungs- und anderen pathologischen Prozessen beeinflussen (Marshall and Hart, 1996, Semin. Cancer Biol. 7, 191).

Die Integrine α₅β₁, α_{IIb}β₃, α₈β₁, αᵥβ₁, αᵥβ₃, αᵥβ₅, αᵥβ₈ und αᵥβ₆ binden alle an die Arg-Gly-Asp (RGD) Peptidsequenz in natürlichen Liganden, wie z.B. Fibronektin oder Vitronektin. Lösliche RGD-haltige Peptide vermögen die Interaktion jedes dieser Integrine mit dem entsprechenden natürlichen Liganden zu inhibieren.

αᵥβ₆ ist ein relativ seltenes Integrin (Busk et al., 1992 J. Biol. Chem. 267(9), 5790; Breuss et al., 1993, J. Histochem. Cytochem. 41(10), 1521), das bei Reparaturvorgängen in Epithelgewebe vermehrt gebildet wird und die natürlichen Matrixmoleküle Fibronectin und Tenascin bevorzugt bindet (Wang et al., 1996, Am. J. Respir. Cell Mol. Biol. 15(5), 664; Huang et al., 1998, J. Cell Sci. 111, 2189). Die physiologischen und pathologischen Funktionen von αᵥβ₆ sind noch nicht genau bekannt, es wird jedoch vermutet, daß dieses Integrin bei physiologischen Vorgängen und Erkrankungen (z. B. Entzündungen, Wundheilung, Tumore), bei denen epitheliale Zellen beteiligt sind, eine wichtige Rolle spielt *(*Breuss et al., 1995, J . Cell Sci. 108, 2241*).* So wird αᵥβ₆ auf Keratinozyten in Wunden exprimiert (Haapasalmi et al., 1996, J. Invest. Dermatol. 106(1), 42; Cass et al., 1998, J. Pediatr. Surg. 33(2), 312), woraus anzunehmen ist, daß neben Wundheilungsprozessen und Entzündungen auch andere pathologische Ereignisse der Haut, wie z. B. Psoriasis, durch Agonisten oder Antagonisten des besagten Integrins beeinflußbar sind.

Weiterhin zeigen Verhornungsstörungen der Haut, sogenannte Leukoplakien, in der Mucosa der Mundhöhle, an den Lippen, der Zunge, sowie den Genitalien erhöhte Expression von Integrin αᵥβ₆, im Gegensatz zu normalem Vergleichsgewebe. Es wurde auch gezeigt, dass die Häufigkeit und die Höhe der Expression von den Leukoplakien, über lichen planus, zum Plattenepithel-Karzinom (squamous cell carcinoma) zunimmt, dass also die Expression von αᵥβ₆ im Zusammenhang mit der malignen Transformation von Leukplakien stehen kann (Hamidi et al., 2000, Br. J. Cancer. 82(8), 1433; Ramos et al., 1997, Int. J. Cancer. 72(2), 369; Thomas et al., 2001, J. Inv. Dermatol. 117(1), 67; Koivisto et al., 2000, Exp. Cell Res. 255(1), 10).

Ferner spielt αᵥβ₆ im Atemwegsepithel eine Rolle (Weinacker et al., 1995, Am. J. Respir. Cell Mol. Biol. 12(5), 547), so daß entsprechende Agonisten / Antagonisten dieses Integrins bei Atemwegserkrankungen, wie Bronchitis, Asthma, Lungenfibrosen und Atemwegstumoren erfolgreich eingesetzt werden könnten (Huang et al., 1998, Am. J. Respir. Cell Mol. Biol. 19(4), 636; Pilewski et al., 1997, Am. J. Physiol. 273, L256; Kaminski et al., 2000, Proc. Natl. Acad. Sci. USA. 97(4), 1778).

Fibrosen sind nicht nur in der Lunge (Bronchien) bekannt, sie werden auch in anderen Organen vorgefunden, so dass das Integrin αᵥβ₆ auch dort eine Rolle spielt; Beispiele sind krankhafte Bindegewebsvermehrungen z.B. der Haut, der Leber (bis zur Zirrhose), der Niere und der Blase, des Herzens und der Bauchspeicheldrüse (Mukoviszidose) (Mutsaers et al., 1997, J. Int. J. Biochem. Cell Biol. 29(1), 5; Dalton, S.L. 1999, J. Am. Acad. Dermatol. 41, 457; Kropf et al., 1991, Z. Med. Laboratoriumsdiagn. 32(3/4), 150; Schnee et al., 2000, Cardiovasc. Res. 46(2), 264; Sime, P.J. 1999 Curr. Opin. Anti-Inflammatory Immunomodulatory Invest. Drugs 1(5), 423; Housset et al., 1999, J. Pathol. Biol. 47(9), 886; Norman et al., 1999, Exp. Nephrol. 7(2), 167; Nahas et al., 1997, Int. J. Biochem. Cell Biol. 29(1), 55).
Es ist ebenfalls bekannt, daß αᵥβ₆ auch im Darmepithel eine Rolle spielt, so daß entsprechende Integrin-Agonisten/-Antagonisten bei der Behandlung von Entzündungen, Tumoren und Wunden des Magen/Darmtraktes Verwendung finden könnten. Es gibt dabei Hinweise, dass das Integrin αᵥβ₆ auch die Sekretion von Matrix-Metalloproteasen beeinflusst, wie z.B. die der Gelatinase B (MMP-9) (Agrez et al., 1994, J.Cell. Biol. 127(2), 547; Niu et al., 1998, Biochem. Biophys. Res. Commun. 249(1), 287).

Die Regelung der MMP-Aktivität (möglicherweise verschiedener MMPs) durch Tumorzellen in Abhängigkeit ihrer Dichte ist auch ein Mechanismus, der den Zellen ermöglicht, beim Wachsen der Tumormasse, sich durch Proteolyse der umgebenden Matrix erneuten Platz für Proliferation und Migration zu verschaffen. Auf diesen Zusammenhang von αᵥβ₆ Expression, Zelldichte und MMP Aktivität ist in der Literatur hingewiesen (Niu et al., 2001, Int. J. Cancer 92(1), 40; Agrez et al., 1999 Int. J. Cancer 81(1), 90; Thomas et al., 2001, J. Inv. Dermatol. 116(6), 898; Thomas et al., 2001 Int. J. Cancer 92(5), 641).

Da das Integrin αᵥβ₆ bei Infektionsvorgängen eine Rolle spielt, können seine Agonisten/Antagonisten bei mikrobiellen Infektionen Verwendung finden (Protozoen, Mikrophyten; Bakterien, Viren, Hefen, Pilze). Beispiele sind für den Coxsackievirus bekannt und für den Befall von Wirtszellen mit dem Virus der Maul-und-Klauenseuche (FMDV), der αᵥβ₃ -abhängig verlaufen, jedoch auch αᵥβ₆ -abhängig erfolgen kann (Agrez et al., 1997, Virology. 239(1), 71; Jackson et al., 2000, J. Virol. 7.4(11); 4949; Miller et al., 2001, J. Virol. 75(9), 4158; Neff et al., 2001, J. Virol. 75(1), 527; Neff et al., 1998, J. Virol. 72(5), 3587; Jackson et al., 1997 J. Virol. 71 (11), 8357).

Auch die Infektion mit HIV (AIDS) ist von αᵥ -Integrinen abhängig, wie schon vor Jahren gezeigt wurde (Ruoslahti et al., WO 92/14755).

Nach neueren Erkenntnissen sekretiert das Bakterium Bacillus anthracis ein Toxin, dass aus 3 Proteinen besteht, von denen eines, das sogenannte PA oder Protective Antigen, an zellmembranständige Rezeptoren bindet, und der dabei beschriebene Rezeptor ATR (Anthrax Toxin Receptor) ein Typ I Membranprotein mit einer extrazellulären Domäne vom Typ von Willebrandt Factor A darstellt. Auch Integrine enthalten solche vWFA Domänen. Sowohl für Integrin αᵥβ₆ ist dies über eine Homologiebetrachtung in der Datenbank Swiss Prot nachvollziehbar (http://www.expasy.ch/cgi-bin/niceprot.pl?P18564; hier Sequenz β₆ (131-371)), als auch für αᵥβ₃ (http://www.expasy.ch/cgi-bin/niceprot.pl?P05106; β₃ (135-377)). Daher können die erfindungsgemäßen Agonisten/Antagonisten auch im Falle von Anthrax (Milzbrand der Lunge, der Haut und des Darmes) genutzt werden (Bradley et al., 2001, Nature 414, 225; Tuckwell, 1999, Biochem. Soc. Trans. 27(6), 835).

Die Abhängigkeit des Befalls von Wirtszellen von deren Adhäsionsrezeptoren wurde für Bakterien ebenso beschrieben, wie für Hefen (Sprosspilzen, Candida) (Kerr, JR. 1999 Medical Microbiology, Manchester Royal Infirmary, UK, MOLECULAR PATHOLOGY, 52(4), 220; Dehio et al., 1998, FEBS LETT. 424(1-2), 84, Stockbauer et al., 1999 Proc. Nat. Acad. Sci. USA. 96(1), 242; Santoni et al., 2001 Microbial Pathogen. 31 (4), 159).

Die Wechselwirkung des Integrins αᵥβ₆ mit TGF-β und und die dadurch erfolgende Aktivierung konnte nachgewiesen werden. (Dalton, SL. 1999, J. Am. Acad. Dermatol 41, 457; Munger et al., 1999 Cell 96, 319).

Es wurde publiziert, daß latentes TGFβ1 (i.e. eine der Pro-Formen) an das Integrin αᵥβ₆ bindet und dann proteolytisch aktiviert wird. Die erfindungsgemäßen Verbindungen könnten die Bindung von TGF-β (Pro-Form, LAP-Peptid, LAP-TGFβ, Latentes TGF) an das Integrin inhibieren, und dadurch könnten Agonisten/Antagonisten des Integrins αᵥβ₆ die Aktivierung von TGF-β1 und anderen Subtypen verhindern. Eine Modulation von TGFβ wäre dadurch ermöglicht.
Bisher wurden 3 humane TGFβ-Isoformen entdeckt wurde, denen eine Rolle bei einer Vielzahl von Wachstums- und Differenzierungprozessen, insbesondere aber bei entzündlichen Prozessen, Fibrosen, Wundheilung, Knochenwachstum, der Modulation von Immunfunktionen, bei Angiogenese und Tumormetastase zugeordnet wird (Rifkin et al., 1993, 70, 177; Hata et al., 1998, Mol. Med. Today 257; Sheppard,D. 2001, Chest. 120(1 Suppl), 49S; Wickstom et al. 1998, Prostate 37, 19). Eine Nutzbarkeit der erfindungsgemäßen Verbindungen als Agonisten/Antagonisten von αᵥβ₆ ist auch in diesen Prozessen zu vermuten.

Eine weitere Arbeit, die die Rolle von αᵥβ₆ bei immunologischen Vorgängen hervorhebt ist, beschreibt den Influx von Neutrophilen nach chemischer Schädigung der Lunge (Miller et al., 2001, J. Histochem. Cytochem. 49(1), 41).

Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569 (1994) beschrieben.

Es bestand somit die Aufgabe, neben den bisher bekannten natürlichen hochmolekularen Liganden und Antikörpern, die therapeutisch und diagnostisch schwer handhabbar sind, potente, spezifische bzw. selektive niedermolekulare peptidische Liganden für das Blutplättchenintegrin GPIIbIIIa und für die αᵥ-Integrine, vorzugsweise für αᵥβ₆ und αᵥβ₃, zu finden, die für die genannten therapeutischen Gebiete aber auch als Diagnostikum oder Reagenz verwendet werden können.

Es wurde gefunden, daß die erfindungsgemäßen peptidischen Verbindungen und ihre Salze Wirkung auf das Blutplättchenintegrin GPIIbIIIa und die αᵥ-Integrine, bevorzugt auf αᵥβ₆- und αᵥβ₃-Integrine oder Zellen ausüben, die diese Integrine tragen, oder, wenn sie an Oberflächen gebunden sind, künstliche Liganden für die Bindung des isolierten GPIIbIIIa oder der isolierten αᵥ-Integrine, bevorzugt αᵥβ₆- und αᵥβ₃-Integrine oder GPIIbIIIa- bzw. αᵥ-vermittelter Funktionen der Zellen darstellen. Vor allem wirken sie als GPIIbIIIa-Integrin, αᵥ-Integrin-, bevorzugterweise αᵥβ₆- und/oder αᵥβ₃-Integrin-Inhibitoren, wobei sie insbesondere die Wechselwirkungen des Rezeptors mit anderen Liganden hemmen, wie z. B. die Bindung von Fibronektin. Diese Wirkung kann z.B. nach der Methode nachgewiesen werden, die von Smith et al. in J. Biol. Chem. 265, 12267-12271 (1990) beschrieben wird.

Weiter wurde gefunden, daß die erfindungsgemäßen Substanzen bei guter Verträglichkeit sehr wertvolle pharmakologische Eigenschaften besitzen und als Arzneimittel eingesetzt werden können. Dies wird weiter unten genauer beschrieben. Diese Eigenschaften bleiben auch erhalten, wenn die Aminosäuren, die nicht in Position X stehen, ausgetauscht werden, und/oder die linearen Verbindungen zyklisiert werden wie in WO 01/00660 und WO 01/05810 beschrieben.

Es wird ferner beschrieben, dass die erfindungsgemäßen peptidischen Verbindungen als Diagnostika zur Detektion und Lokalisierung von pathologischen Zuständen im epithelialen System *in vivo* verwendet werden können, wenn sie mit entsprechenden Markermolekülen nach dem Stand der Technik ausgestattet sind. Hierzu eignet sich z.B. Biotin (z.B. zu Affinitätszwecken), oder verschiedene Fluoreszenzlabel oder Radioisotopen-Komplexe für die Diagnostik und bildgebende Verfahren. Die erfindungsgemäßen Verbindungen können auch Ankerfunktionen tragen, um kovalent auf Oberflächen von Werkstücken, wie z.B. Bioimplantaten, Mikrotiterplatten oder Partikeln gekuppelt zu werden.

Die Erfindung beschreibt auch Kombinationen mit mindestens einem anderen Wirkstoff und/oder Konjugate mit anderen Wirkstoffen, wie zytotoxischen Wirkstoffen sowie Konjugate mit Radiomarkern für Röntgentherapie oder PET Diagnose aber auch Fusionsproteine mit Markerproteinen wie GFP oder Antikörpern, oder therapeutischen Proteinen wie IL-2.

Im Rahmen der vorliegenden Erfindung bedeutet Alkyl einen linearen oder verzweigten oder zyklischen Alkylrest mit 1, 2, 3, 4, 5, 6, 7, 8, 9, 10, 11, 12, 13, 14, 15, 16, 17 oder 18 C-Atomen, die substituiert, z. B. halogeniert, hydroxyliert, animiert, teilweise ungesättigt oder von Heteroatomen wie N, S oder O unterbrochen sein können. Wenn ein Alkylrest mit Halogen substituiert ist, weist er vorzugsweise, abhängig von der Anzahl der Kohlenstoffatome des Alkylrests 1, 2, 3, 4 oder 5 Halogenatome auf. So kann beispielsweise eine Methylgruppe 1-, 2- oder 3-fach mit Halogen substituiert sein, und eine Ethylgruppe 1-, 2-, 3-, 4- oder 5-fach mit Halogen substituiert sein. Bevorzugt steht Alkyl für Methyl, Ethyl, Trifluormethyl, Pentafluorethyl oder Propyl, besonders bevorzugt für Isopropyl, Butyl, Isobutyl, sek.-Butyl oder tert.-Butyl, aber auch für n-Pentyl, neo-Pentyl oder Isopentyl.

Der Ausdruck "Aryl" bedeutet einen unsubstituierten oder einfach oder mehrfach substituierten aromatischen mono-, bi- oder tricyclischen Kohlenwasserstoffrest wie zum Beispiel einen Benzolring oder Anthracen-, Phenanthren- oder Napthalen-Ringsysteme. Beispiele für geeignete Substituenten umfassen NO₂- F-, Cl-, Br-, J-, HO-, H₂N-, R³HN-, (R³)₂N-, Alkyl-, Alkyl-O-, CF₃-O-, Alkyl-CO-, Aryl-, Aryl-O-, Aryl-CO-, Aryl-CONH-, ArylSO₂-, ArylSO₂-HN-, wobei die Substituenten unabhängig voneinander 0 bis 5 mal vorkommen können.

Der Ausdruck "Het" bedeutet einen unsubstituierten oder einfach oder mehrfach substituierten, gesättigten, ungesättigten oder aromatischen mono-, bi- oder tricyclischen heterocyclischen Rest. Als Heteroatome können S, N oder O ein- bis dreimal vorkommen. Beispiele für geeignete Substituenten umfassen NO₂-, F-, Cl-, Br-, J-, HO-, H₂N-, R³HN-, (R³)₂N-, Alkyl-, Alkyl-O-, CF₃-O-, Alkyl-CO-, Aryl-, Aryl-O-, Aryl-CO-, Aryl-CONH-, ArylSO₂-, ArylSO₂-HN-, wobei die Substituenten unabhängig voneinander 0 bis 5 mal vorkommen können.

Der Ausdruck "Aralkyl" bedeutet vorzugsweise einen Arylrest wie obenstehend definiert, verbunden mit einem Alkylrest wie obenstehend definiert. Beispiele für geeignete Aralkylreste umfassen, sind aber nicht beschränkt auf, Benzyl, Phenylpropyl, Phenylbutyl und dergleichen.

Der Ausdruck αᵥ-Integrine umfasst in der vorliegenden Erfindung die Integrine, αᵥβ₁, αᵥβ₃, αᵥβ₅, αᵥβ₆ und αᵥβ₈, bevorzugt jedoch das αᵥβ₆- und αᵥβ₃-Integrin.

Gegenstand der Erfindung sind insbesondere peptidische Verbindungen ausgewählt aus der Gruppe der Formeln Ia) - I n)
I a) Ac-Arg-Dap(Psa)-Asp-Leu-Asp-Ser-Leu-Arg-NH₂,
I b) Ac-Arg-Dap(F5-PSA)-Asp-Leu-Asp-Ser-Leu-Arg-NH₂,
I c) Ac-Arg-Dap(2-NO₂-PSA)-Asp-Leu-Asp-Ser-Leu-Arg-NH₂,
I d) Ac-Arg- Dap(4-NO₂-PSA)-Asp-Leu-Asp-Ser-Leu-Arg-NH₂,
I e) Ac-Arg- Dap(2,4-NO₂-PSA)-Asp-Leu-Asp-Ser-Leu-Arg-NH₂,
I f) Ac-Arg-Dap(6-OMe-PSA)-Asp-Leu-Asp-Ser-Leu-Arg-NH₂,
I g) Ac-Arg-Dap(2-CF₃-PSA)-Asp-Leu-Asp-Ser-Leu-Arg-NH₂,
I h) Ac-Arg-Dap(3-CF₃-PSA)-Asp-Leu-Asp-Ser-Leu-Arg-NH₂,
I i ) Ac-Arg-Dap(Me₅-PSA)-Asp-Leu-Asp-Ser-Leu-Arg-NH₂,
I j ) Ac-Arg-Dap(4-tBu-PSA)-Asp-Leu-Asp-Ser-Leu-Arg-NH₂,
I k) Ac-Arg-Dap(BSA)-Asp-Leu-Asp-Ser-Leu-Arg-NH₂,
I l)Ac-Arg-Dap(1-Nap)-Asp-Leu-Asp-Ser-Leu-Arg-NH₂,
I m) Ac-Arg-Dap(2-Nap)-Asp-Leu-Asp-Ser-Leu-Arg-NH₂,
I n) Ac-Arg-Dap(4-Ph-Psa)-Asp-Leu-Asp-Ser-Leu-Arg-NH₂,
sowie deren pharmazeutisch verwendbaren Prodrugs, Derivate, Solvate und Stereoisomere, sowie deren Salze, einschließlich deren Mischungen in allen Verhältnissen, wobei die in den Formeln I a) - I n) in Klammern verwendeten Abkürzungen für folgende Reste stehen:
- Psa: Phenylsulfonylrest
- F5-PSA: Pentafluorphenylsulfonylrest
- 2-NO₂-PSA: 2-Nitro-Phenylsulfonylrest
- 4-NO₂-PSA: 4-Nitro-Phenylsulfonylrest
- 2,4-NO₂-PSA: 2,4-Dinitro-Phenylsulfonylrest
- 6-OMe-PSA: 6-Methoxy-Phenylsulfonylrest
- 2-CF₃-PSA: 2-Trifluormethyl-Phenylsulfonylrest
- 3-CF₃-PSA: 3-Trifluormethyl-Phenylsulfonylrest
- Me₅-PSA: Pentamethylphenylsulfonylrest
- 4-tBu-PSA: 4-tert-Butyl-Phenylsulfonylrest
- Bsa: Benzylsulfonylrest
- 1-Nap: 1-Naphthylsulfonylrest
- 2-Nap: 2-Naphthylsulfonylrest
- 4-Ph-Psa: 4-Phenyl-Phenylsolfonylrest

Die vor- und nachstehend aufgeführten Abkürzungen von Aminosäureresten stehen für die Reste folgender Aminosäuren:
- Asp oder D: Asparaginsäure
- Arg oder R: Arginin
- Dap: 2,3-Diaminopropionsäure
- Leu oder L: Leucin
- Ser oder S: Serin
- Thr oder T: Threonin

Ferner bedeuten vor- und nachstehend:
- Ac: Acetyl
- BOC: tert.-Butoxycarbonyl
- BSA: Bovine Serum Albumin
- CBZ oder Z: Benzyloxycarbonyl
- DCCI: Dicyclohexylcarbodümid
- DCM: Dichlormethan
- DIEA: Diisopropylamin
- DMA: N,N-Dimethylacetamid
- DMF: Dimethylformamid
- EDCI: N-Ethyl-N,N'-(dimethylaminopropyl)-carbodiimid
- Et: Ethyl
- FCA: Fluoresceincarbonsäure
- FITC: Fluoresceinisothiocyanat
- Fmoc: 9-Fluorenylmethoxycarbonyl
- Fmoc-Dap(ivDde): N-α-Fmoc-N-γ-1-(4,4-dimethyl-2,6-dioxocyclohex-1-yliden)-3-methylbutyldiaminopropionsäure
- FTH: Fluoresceinthioharnstoff
- HOBt: 1-Hydroxybenzotriazol
- Me: Methyl
- MBHA: 4-Methyl-benzhydrylamin
- Mtr: 4-Methoxy-2,3,6-trimethylphenyl-sulfonyl
- NMP: N-Methylpyrrolidon
- HONSu: N-Hydroxysuccinimid
- OBut: tert.-Butylester
- Oct: Octanoyl
- OMe: Methylester
- OEt: Ethylester
- Pbf: 2,2,4,6,7-Pentamethyl-dihydrobenzofuran-5-sulfonyl
- Pmc: 2,2,5,7,8-Pentamethylchroman-6-sulfonyl
- POA: Phenoxyacetyl
- Sal: Salicyloyl
- TBS++: Tris buffered Saline mit 2-wertigen Kationen
- TBSA: TBS+BSA
- TBTU: 2-(1 H-Benzotriazol-1-yl)-1,1,3-tetramethyluronium tetrafluoroborat
- TFA: Trifluoressigsäure
- TIS: Triisopropylamin
- Trt: Trityl (Triphenylmethyl).

Sofern die vorstehend genannten Aminosäuren in mehreren enantiomeren Formen auftreten können, so sind vor- und nachstehend, alle diese Formen und auch ihre Gemische (z. B. die DL-Formen) eingeschlossen. Ferner können die Aminosäuren mit entsprechenden an sich bekannten Schutzgruppen versehen sein.

In die erfindungsgemäßen Verbindungen sind auch sogenannte Prodrug-Derivate eingeschlossen, d. h. mit Alkyl- oder Acylgruppen, Zuckern oder Oligopeptiden abgewandelte Verbindungen, die im Organismus rasch zu den wirksamen erfindungsgemäßen Verbindungen gespalten werden. Es werden auch bioabbaubare Polymerderivate der erfindungsgemäßen Verbindungen erwähnt wie dies z. B. in Int. J. Pharm. 115, 61-67 (1995) beschrieben ist.

Die genannten Aminosäuren und Aminosäurereste, wie z.B. die NH-Funktionen oder auch die C-terminale Amidfunktion können auch derivatisiert sein, wobei die N-Methyl-, N-Ethyl-, N-Propyl-, N-Benzyl- oder C_{α}-Methylderivate ausgewählt werden. Weiterhin werden Derivate von Asp, insbesondere solche mit Methyl-, Ethyl, Propyl, Butyl, tert.-Butyl, Neopehtyl- oder Benzylester der Seitenkettencarboxygruppen, ferner auch Derivate von Arg, das an der -NH-C(=NH)-NH₂ -Gruppe mit einem Acetyl-, Benzoyl-, Methoxycarbonyl- oder Ethoxycarbonylrest substituiert sein kann, beschrieben.

In den erfindungsgemäßen Verbindungen sind neben den Verbindungen der Formel I, die N-terminal eine Acetylgruppe tragen auch solche Verbindungen eingeschlossen, in denen Ac durch eine andere Acylfunktion ersetzt ist, ausgewäht aus Propionyl, Butyryl und Benzoyl.

Ferner werden mit den erfindungsgemäßen Verbindungen auch Derivate beschrieben, welche aus den eigentlichen erfindungsgemäßen Verbindungen bestehen, die mit bekannten Marker-Molekülen derivatisiert sind, die es ermöglichen, die Peptide leicht nachzuweisen. Beispiele für solche Derivate sind radioaktiv markierte, biotinylierte oder fluoreszenzmarkierte Peptide.

Fluoreszierender Farbstoffrest bedeutet vorzugsweise 7-Acetoxycoumarin-3-yl, Fluorescein-5-(und/oder 6-)yl, 2',7'-DichlorflÜorescein-5-(und 6-)yl, Dihydrotetramethylrosamin-4-yl, Tetramethylrhodamin-5-(ünd/oder 6-)yl, 4,4-Difluor-5,7-dimethyl-4-bora-3a,4a-diaza-s-indacen-3-ethyl oder 4,4-Difluor-5,7-diphenyl-4-bora-3a;4a-diaza-s-indacen-3-ethyl.

Geeignete funktionalisierte fluoreszierende Farbstoffreste, die als Reagenzien zur Herstellung der erfindungsgemäßen Verbindungen der Formel I dienen können, sind z. B. beschrieben in "Handbook of Fluorescent Probes and Research Chemicals, 5th Edition, 1992-1994, by R.P. Haughland, Molecular Probes, Inc.".

Im allgemeinen sind die erfindungsgemäßen Peptide linear, sie können aber auch zyklisiert werden. Die Erfindung umfaßt nicht nur die genannten Peptide sondern auch Mischungen und Zubereitungen, welche neben diesen erfindungsgemäßen Verbindungen auch andere pharmakologische Wirkstoffe oder Adjuvantien enthalten, die die primäre pharmakologische Wirkung der erfindungsgemäßen Peptide in gewünschter Weise beinflussen können.

Die erfindungsgemäßen Verbindungen und auch die Ausgangsstoffe zu ihrer Herstellung werden im übrigen nach an sich bekannten und häufig eingesetzten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten Varianten Gebrauch machen.

Vorzugsweise können die erfindungsgemäßen Peptide mittels Festphasensynthese und nachfolgender Abspaltung und Reinigung hergestellt werden, wie dies z.B. von *Jonczyk und Meienhofer* (Peptides, Proc. 8th Am. Pept. Symp., Eds. V. Hruby und .D.H.Rich, Pierce Comp. III, p. 73-77, 1983, oder Angew. Chem. 104, 1992, 375) oder gemäß Merrifield (J. Am. Chem. Soc. 94, 1972, 3102) beschrieben wurde.

Die erfindungsgemäßen Peptide können an fester Phase (manuell oder in einem Syntheseautomaten) in einer Fmoc-Strategie mit säurelabilen Seitenschutzgruppen hergestellt und mittels RP-HPLC gereinigt werden. Die Peakeinheitlichkeit kann durch RP-HPLC und die Substanzidentität mittels FAB-MS gemessen werden.

Im übrigen können die Peptide nach üblichen Methoden der Aminosäure- und Peptidsynthese hergestellt werden, wie dies z. B. aus Novabiochem - 1999 Catalog & Peptide Synthesis Handbook der Calbiochem-Novabiochem GmbH, D-65796 Bad Soden, aus zahlreichen Standardwerken und publizierten Patentanmeldungen bekannt ist.

Es können auch schrittweise Kupplungen und Fragmentkondensationen genutzt werden. Unterschiedliche N-terminale, C-terminale und Seitenschutzgruppen können Verwendung finden, die bevorzugt orthogonal spaltbar ausgewählt werden. Kupplungsschritte können mit unterschiedlichen Kondensationsreagenzien wie Carbodiimiden, Carbodiimidazol, solchen des Uronium-Typs wie TBTU, gemischten Anhydrid-Methoden, sowie Säurehalogenid- oder Aktivester-Methoden durchgeführt werden.
Eine Cyclisierung eines linearen Vorläufermoleküls mit Seitenschutzgruppen ist ebenfalls mit solchen Kondensationsreaktionen durchführbar, wie z. B. in DE 43 10 643 oder in Houben-Weyl, I.c., Band 15/II, Seiten 1 bis 806 (1974) beschrieben.

Bei der Festphasenpeptidsynthese sind unterschiedliche Harze und Ankerfunktionen nutzbar. Harze können z.B. auf Polystyrol oder Polyacrylamid basieren, Ankerfunktionen wie Wang, o-Chlortrityl sind zur Herstellung von Peptidsäuren, Aminoxanthenoxy-Anker z.B. zur Herstellung von Paptidamiden nutzbar. (vgl ; Principles of Peptide Synthesis, ed. M. Bodansky, Springer Verlag Berlin 1984; Houben Weyl Methoden der organischen Chemie, Georg Thieme Verlag, Stuttgart; Calbiochem/Novabiochem Catalogue and Synthesis Handbook 1999, Synthesis Notes; Peptide Synthesis Protocols eds. M.W. Pennington and B.M. Dunn in Methods in Molecular Biology Vol 35, Humana Press Totowa N.J. 1994)

Biotinylierte oder fluoreszenzmarkierte Peptide / Proteine können ebenfalls nach Standardmethoden hergestellt werden (z.B. E.A. Bayer and M. Wilchek in Methods of Biochemical Analysis Vol 26 The Use of the Avidin-Biotin Complex as a Tool in Molecular Biology; und Handbook of Fluorescent Probes and Research Chemicals, 6th Edition, 1996, by R.P. Haugland, Molecular Probes, Inc.; oder auch WO 97/14716).

Selbstverständlich können die erfindungsgemäßen Peptide auch durch Solvolyse, insbesondere Hydrolyse, oder durch Hydrogenolyse ihrer funktionellen Derivate freigesetzt werden. Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe oder die anstelle des H-Atoms einer Hydroxygruppe eine Hydroxyschutzgruppe tragen. Entsprechendes gilt für Carbonsäuren, die durch Substitution ihrer -CO-OH Hydroxyfunktion mittels einer Schutzgruppe, z.B. als Ester geschützt werden können.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Das In-Freiheit-Setzen der Verbindungen aus ihren funktionellen Derivaten gelingt - je nach der benutzten Schutzgruppe - z. B. mit starken Säuren, zweckmäßig mit TFA, HBr oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden.

Typische Schutzgruppen für N-Termini und für seitenständige Aminogruppen sind Z, BOC, Fmoc, solche für C-Termini oder die Asp-Seitenketten sind O-prim.-Alkyl (z.B. OMe oder OEt), O-tert.-Alkyl (z.B. OBut) oder OBenzyl. Für die Guanidinofunktion des Arg ist z.B. Z, BOC, NO₂, Mtr, Pmc oder Pbf geeignet. Alkoholische Funktionen können durch tert.-Alkylreste, Benzylrest oder Tritylgruppen geschützt sein.

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol, Ether wie THF oder Dioxan oder Amide wie DMF, DMA oder NMP. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Wie bereits erwähnt, sind die Salze der Verbindungen nach Formel I und die Salze der verwendbaren Prodrugs, Derivate, Solvate und Stereoisomere der Verbindungen nach Formel I ebenfalls umfasst. Dies können nach Standardmethoden hergestellt werden. So kann eine Base einer erfindungsgemäßen Verbindung mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, lsonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können, zur Isolierung und /oder Aufreinigung der erfindungsgemäßen Verbindungen verwendet werden. Andererseits kann eine Säure der erfindungsgemäßen Verbindungen durch Umsetzung mit einer Base in eines ihrer physiologisch unbedenklichen Metall- oder Ammoniumsalze übergeführt werden. Als Salze kommen dabei insbesondere die Natrium-, Kalium, Magnesium-, Calcium- und Ammoniumsalze in Betracht, ferner substituierte Ammoniumsalze, z. B. die Dimethyl-, Diethyl- oder Diisopropylammoniumsalze, Monoethanol-, Diethanol- oder Düsopropylammoniumsalze, Cyclohexyl-, Dicyclohexyl-ammoniumsalze, Dibenzylethylendiammoniumsalze, weiterhin z. B. Salze mit Arginin oder Lysin.

Die erfindungsgemäßen peptidischen Verbindungen können, wie bereits erwähnt, als Arzneimittelwirkstoffe in der Human- und Veterinärmedizin zur Prophylaxe und/oder Therapie eingesetzt werden. Besonders hervorzuheben sind hierbei Erkrankungen des Kreislaufs, Lungenembolie, Thrombose, insbesondere tiefe Venenthrombose, Herzinfarkt, Arteriosklerose, Aneurysma dissecans, vorübergehende ischämische Anfälle, Apoplex, Angina pectoris, insbesondere instabile Angina pectoris, krankhafte Bindegewebsvermehrungen in Organen oder Fibrosen, insbesondere Lungenfibrose, aber auch Mucoviszidose, Hautfibrose, Leberfibrosen, Leberzirrhose, Blasenausgangsfibrosen, Nierenfibrose, Herzfibrose, infantile endocardiale Fibrose, Pankreasfibrose, Verhornungsstörungen der Haut, Leukoplakien, lichen planus, und Plattenepithelkarzinome, Tumorerkrankungen, wie Tumorentwicklung, Tumorangiogenese oder Tumormetastasierung, dabei soliden Tumoren und solchen des Blutes oder Immunsystems, z.B. Tumore der Haut, Plattenepithelkarzinome, Tumore der Blutgefäße, des Magendarmtraktes, der Lunge, der Brust, der Leber, der Niere, der Milz, der Bauchspeicheldrüse, des Gehirns, der Hoden, des Ovars, der Gebärmutter, der Scheide, der Muskulatur, der Knochen, und solchen des Hals- und Kopf-Bereiches, osteolytische Krankheiten, wie Osteoporose, Hyperparathyroidismus, Morbus Paget, maligne Hypercalcämie, Entzündungen, ophthalmologische Krankheiten wie z.B diabetischer Retinopathy, makulare Degeneration, Myopia, Corneatransplantation, rubeotisches Glaukom, okulare Histoplasmose, rheumatische Arthritis, Osteoarthritis, ulcerative Colitis, Morbus Crohn, Atherosklerose, Psoriasis, Restenose, insbesondere nach Angioplastie, Multiple Sklerose, bei akutem Nierenversagen und bei der Wundheilung zur Unterstützung des Heilungsprozesses.

Gegenstand der Erfindung sind demgemäß peptidische Verbindungen der oben und unten sowie in den Ansprüchen definierten Formeln einschließlich ihrer physiologisch unbedenklichen Salze als Arzneimittel, Diagnostika oder Reagenzien.

Gegenstand der Erfindung sind insbesondere entsprechende Arzneimittel als Inhibitoren zur Bekämpfung von den oben genannten Erkrankungen, bei denen GPIIbIIIa, αᵥ-Integrine, bevorzugt αᵥβ₆- und/oder αᵥβ₃-Integrin eine Rolle spielen, insbesondere also bei Erkrankungen des Kreislaufs, krankhaften Bindegewebsvermehr-ungen in Organen und Fibrosen, Verhornungsstörungen, Tumorerkrankungen, osteolytischen Krankheiten, pathologisch angiogenen Erkrankungen, Infektionen sowie zur Beeinflussung von Wundheilungsprozessen.

Gegenstand der Erfindung sind auch entsprechende pharmazeutische Zubereitungen, welche mindestens eine Verbindung der Formel I oder ein physiologisch unbedenkliches Prodrug, Derivat, Solvat oder Stereoisomer oder ein Salz davon sowie gegebenenfalls Träger- und/oder Hilfsstoffe enthalten.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate und Stereoisomere und deren Salze, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere und Salzen davon, einschließlich deren Mischungen in allen Verhältnissen, und einer wirksamen Menge eines weiteren Arzneimittelwirkstoffs gelöst oder in lyophilisierter Form vorliegt.

Ferner ist Gegenstand der Erfindung die Verwendung der Verbindungen nach Formel I sowie die pharmazeutisch verwendbaren Prodrugs, Derivate, Solvate und Stereoisomere der Verbindungen nach Formel I sowie die Salze davon, wobei deren Mischungen in allen Verhältnissen umfasst sind, zur Herstellung eines Arzneimittels zur Bekämpfung von den oben genannten Erkrankungen, bei denen GPllbllla, αᵥ-Integrine, bevorzugt αᵥβ₆- und/oder αᵥβ₃-Integrin eine Rolle spielen, insbesondere also bei Erkrankungen des Kreislaufs, krankhaften Bindegewebsvermehrungen in Organen und Fibrosen, Verhornungsstörungen, Tumorerkrankungen, osteolytischen Krankheiten, pathologisch angiogenen Erkrankungen, Infektionen sowie zur Beeinflussung von Wundheilungsprozessen.

Die erfindungsgemäßen Arzneimittel bzw. sie enthaltende pharmazeutische Zubereitungen können in der Human- oder Veterinärmedizin verwendet werden. Sie können lokal oder systemisch, oral, intravenös, intraperitoneal, intramuskulär, subkutan, transdermal, nasal, buccal oder iontophoretisch gegeben werden, das schließt Formulierungen in Suspensionen, Emulsionen oder Lösungen, Liposomen, Salben, Pasten, bioabbaubaren Polymeren oder als Nanopartikel, Tabletten, Kapseln oder Pillen, Granulate oder Puder, als Aerosol zum Inhalieren, als intranasale Tropfen oder Sprays ein. Auch eine Kombination mit anderen Techniken, wie Chirurgie, Bestrahlung, Diagnose, Radiotherapie, photodynamischer Therapie und Gentherapie, sowie mit anderen Medikamenten ist möglich. Solche Medikamente können z.B. aus den Gebieten Herzkreislauf, Zentrales Nervensystem, oder der Onkologie stammen. Es können Antitumormittel sein, wie Angiogeneseinhibitoren oder Cytostatika, Chemotherapeutika der Gruppen alkylierende Agenzien, Antibiotika, Antimetaboliten, Biologika und Immunmodulatoren, Hormone und deren Antagonisten, Senfgasderivaten, Alkaloiden und anderen, wobei diese Substanzen niedermolekular und hochmolekular sein können. Es können Lipide, Kohlehydrate, Proteine sein. Es fallen unter anderem Zytokine, Toxine, Fusionsproteine, Monoklonale Antikörper und Vaccine darunter.

Als Trägerstoffe für die erfindungsgemässen pharmazeutischen Zubereitungen kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder eine oder mehrere weitere Wirkstoffe enthalten, z. B. ein oder mehrere Vitamine.

Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe 'üblicher Inhatatoren verabreicht werden.

Die erfindungsgemäßen Substanzen können in der Regel in Analogie zu anderen bekannten, im Handel befindlichen Peptiden (z.B. beschrieben in der US-A-4 472 305) verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 20 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die neuen Verbindungen der Formel I in der analytischen Biologie und Molekularbiologie verwendet werden.
Die Verbindungen der Formel I, die einen fluoreszierenden Farbstoffrest enthalten, können als diagnostische Marker in der FACS (Fluorescence Activated Cell Sorter)-Technik und Fluoreszenz-Mikroskopie in vitro und in vivo verwendet werden.

Der Einsatz von markierten Verbindungen in der Fluoreszenz-Mikroskopie ist z. B. beschrieben von Y.-L. Wang und D. L. Taylor in "Fluorescence Microscopy of Living Cells in Culture, Part A + B, Academic Press, Inc. 1989". Ein weiteres Beispiel für die Anwendung in vivo ist bei M. Dellian et al. Am. J. Pathol. 149, 59-71 (1996) zu entnehmen.

Die erfindungsgemäßen Verbindungen können auch als Integrinliganden zur Herstellung von Säulen für die Affinitätschromatographie zur Reindarstellung von Integrinen verwendet werden. Der Komplex aus einem Avidinderivatisierten Trägermaterial, z.B. Sepharose und den neuen Verbindungen wird nach an sich bekannten Methoden (z.B. E.A. Bayer and M. Wilchek in Methods of Biochemical Analysis Vol 26 The Use of the Avidin-Biotin Complex as a Tool in Molecular Biology) gebildet. Als polymere Trägermaterialien eignen sich dabei die an sich in der Peptidchemie bekannten polymeren festen Phasen mit vorzugsweise hydrophilen Eigenschaften, beispielsweise quervernetzte Polyzucker wie Cellulose, Sepharose oder Sephadex^{R}, Acrylamide, Polymer auf Polyethylenglykolbasis oder Tentakelpolymere^{R}.

### Beispiele:

Vor- und nachstehend sind alle Temperaturen in °C angegeben.

Die HPLC-Analysen (Retentionszeit Rt) erfolgten in den folgenden Systemen:
Säule 5 µm LichroSpher 60 RP-Select B (250-4), mit einem 50-minütigen Gradienten von 0 bis 80% Acetonitril in Wasser / 0,1% Trifluoressigsäure, bei 1 ml/min Fluss und Detektion bei 215 nm.
Massenspektrometrie (MS): EI (Elektroneristoß-lonisation) M⁺
   FAB (Fast Atom Bombardment) (M+H)⁺

### Beispiel 1

### Synthese von Ac-Arg-Dap-Asp-Leu-Asp-Ser-Leu-Arg-NH₂

a) 1 g Amino-Xanthenylharz (Novabiochem) wird mit Fmoc-Arg(Pbf) / HOBt / DIC in 10 ml DMF gequollen und 2 h bei RT geschüttelt. Zur Aufarbeitung wurde die feste Phase abfiltriert und je 3 mal mit Dichlormethan, DMF, Dichlormethan und Methanol gewaschen und am Vakuum getrocknet.
b) Die feste Phase wird mit DMF suspendiert und anschießend mit einer 50 %igen Lösung von Piperidin in DMF versetzt und 15 min bei RT geschüttelt. Anschließend wird von der festen Phase abfiltriert und die gleiche Vorgehensweise zweimal wiederholt. Abschließend wird die feste Phase je dreimal mit DMF, Dichlormethan und Methanol gewaschen und am Vakuum bei RT getrocknet.
c) Das Arg(Pbf)-NH-Xanthenyl-Harz wird nacheinander der Prozedur a und b für die Aminosäurederivate 1. Fmoc-Leu, Fmoc-Ser(But), Fmoc-Asp(OBut), Fmoc-Leu, Fmoc-Asp(OBut), Fmoc-Dap(Boc), und 7.
   Fmoc-Arg(Pbf) unterworfen.
   Dabei erhält man Arg(Pbf)-Dap(Boc)-Asp(OBut)-Leu-Asp(OBut)-Ser(But)-Leu-Arg(Pbf)-NH-Xanthenyl-Harz.
d) Das Peptidylharz wird in 10 ml DMF / Pyridin / Acetanhydrid (15 : 3 : 2 vol) gequollen und nach 15 min bei RT abfiltriert. Zur Aufarbeitung wird je dreimal mit DMF, Dichlormethan und Methanol gewaschen und über Nacht im Vakuum getrocknet.
   Man erhält dadurch Ac-Arg(Pbf)-Dap(Boc)-Asp(OBut)-Leu-Asp(OBut)-Ser(But)-Leu-Arg(Pbf)-NH-Xanthenyl-Harz.
e) Das N-terminal acetylierte, seitenkettengeschützte Peptidylharz wird mit 20 ml einer 98 %igen Lösung von TFA in Wasser versetzt und 3 h bei RT geschüttelt. Die feste Phase wird durch Filtration entfernt und das Filtrat im Vakuum bei 37 C zur Trockene eingeengt und nach Aufnehmen mit Wasser gefriergetrocknet.
   Es resultiert das gewünschte Produkt Ac-Arg-Dap-Asp-Leu-Asp-Ser-Leu-Arg-NH₂ als flockiger Feststoff.
f) Eine Reingung des Rohproduktes aus 1e) erzielt man an durch RP-HPLC an Lichroprep RP18 in Wasser / 0.1 %TFA mit einem Gradienten von 1-80 % 2-Propanol. Das gewünschte Produkt fällt in hoher Reinheit an und hat die erwartete Masse von (M+H)+ = 1001 g/mol.

### Beispiel 2

### Synthese von Ac-Ar-g-Dap(Psa)-Asp-Leu-Asp-Ser-Leu-Arg-NH₂

Fmoc wurde vom Asp(OBut)-Leu-Asp(OBut)-Ser(But)-Leu-Arg(Pbf)-NH-Xanthenyl-Harz abgespalten und Fmoc-Dap(ivDde) (von Novabiochem) in Doppelkupplung (2/1 Äqu.; 20/40 min) gekuppelt. Nach Capping und Waschen wurde mit Morpholin/DMF (1:1) die Fmoc-Schutzgruppe abgespalten (100 ml in 25 min) und auf die eben beschriebene Weise Fmoc-Arg(Pbf) gekuppelt.

Fmoc wurde normal abgespalten, es wurde gecappt (acetyliert) und gewaschen. Der Kaiser-Test war negativ. Es wurde 10 min (10 ml/min) mit 2 % Hydrazin in DMF die ivDde-Schutzgruppe abgespalten: der Kaiser-Test war positiv. Es wurde gewaschen und getrocknet; die Ausbeute war 2,19 g.

Das teilweise seitengeschützte Ac-Arg(Pbf)-Dap-Asp(OBut)-Leu-Asp(OBut)-Ser(But)-Leu-Arg(Pbf)-NH-Xanthenyl-Harz wurde in 16 Aliquote von 130 mg geteilt, die kurz in DCM gequollen wurden.

Das entsprechende Sulfonylchlorid wurde im Eppendorfgefäß in 1,5 ml DCM gelöst und 5 Äqu. DIEA zugegeben. Die Lösungen wurden zum Harz gegeben. Wenn nach 1 h der Kaiser-Test noch leicht positiv war, wurde abfiltriert, gewaschen und erneut auf die selbe Art gekuppelt. Es wurde gründlich mit DMF, DCM und iso-Propanol gewaschen und getrocknet.

Durch 2 stündige Abspaltung mit je 6 ml TFA/Wasser/TIS (94/3/3) Filtration und Reinigung per RP-HPLC erhält man die freien Peptidderivate; z.B. bei Verwendung von Phenylsulfonylchlorid das Produkt Ac-Arg-Dap(Psa)-Asp-Leu-Asp-Ser-Leu-Arg-NH₂.

### Beispiel 3

Analog Beispiel 2 wurden u. a. folgende Produkte hergestellt, indem die Aminosäure X in Position 2 des Peptides Ac-Arg-X-Asp-Leu-Asp-Ser-Leu-Arg-NH2, durch Einsatz der entsprechenden Fmoc-Aminosäurederivate an Stelle von Fmoc-Dap(ivDde), und den entsprechenden Acylierungsreagenzien an Stelle von Phenylsulfonylchlorid erhalten werden konnte.
Sie sind auch durch den Einsatz der vorgefertigten Aminosäurederivate wie Fmoc-Dap(SO₂Ph)-OH (und die Analogen) in der Peptidsynthese darstellbar.

| Sequenz | Rt[min] HPLC | Summenformel | M+H+ g/mol ber. | M+H+ g/mol gef |
|---|---|---|---|---|
| Ac-R-Dap(Psa)-DLDSLR-NH2 | 15,78^{D} | C₄₆H₇₆N₁₆O₁₆S | 1141,5423 | 571(2H+) |
| Ac-R-Dap(F5-Psa)-DLDSLR-NH2 | 18,14^{D} | C₄₆H₇₁F₅N₁₆O₁₆S | 1231,4951 | 616(2H+) |
| Ac-R-Dap(2-N02-Psa)-DLDSLR-NH2 | 15,90^{D} | C₄₆H₇₅N₁₇O₁₈S | 1186;5273 | 594(2H+) |
| Ac-R-Dap(4-NO2-Psa)-DLDSLR-NH2 | 16,50^{D} | C₄₆H₇₅N₁₇O₁₈S | 1186,5273 | 594(2H+) |
| Ac-R-Dap(2,4-NO2-Psa)-DLDSLR-NH2 | 17,53^{D} | C₄₆H₇₄N₁₈O₂₀S | 1231,5124 | 616(2H+) |
| Ac-R-Dap(6-OMe-Psa)-DLDSLR-NH2 | 15,49^{D} | C₄₇H₇₈N₁₆O₁₇S | 1171,5528 | 586(2H+) |
| Ac-R-Dap(2-CF3-Psa)-DLDSLR-NH2 | 16,86^{D} | C₄₇H₇₅F₃N₁₆O₁₆S | 1209,5297 | 605(2H+) |
| Ac-R-Dap(3-CF3-Psa)-DLDSLR-NH2 | 16.00^{D} | C₄₇H₇₅N₁₆O₁₆S | 1209,5297 | 605(2H+) |
| Ac-R-Dap(Me5-Psa)-DLDSLR-NH2 | 19,60^{D} | C₅₁H₈₆N₁₆O₁₆S | 1211,6205 | 606(2H+) |
| Ac-R-Dap(4-tBu-Psa)-DLDSLR-NH2 | 19,87^{D} | C₅₀H₈₄N₁₆O₁₆S | 1197,6049 | 599(2H+) |
| Ac-R-Dap(Bsa)-DLDSLR-NH2 | 15,61^{D} | C₄₇H₇₈N₁₆O₁₆S | 1155,5579 | |
| Ac-R-Dap(1-Nap)-DLDSLR-NH2 | 17,60^{D} | C₅₀H₇₈N₁₆O₁₆S | 1191,5579 | 596(2H+) |
| Ac-R-Dap(2-Nap)-DLDSLR-NH2 | 17,99^{D} | C₅₀H₇₈N₁₆O₁₆S | 1191,5579 | 596(2H+) |
| Ac-R-Dap(4-Ph-Psa)-DLDSLR-NH2 | 19,65^{D} | C₅₂H₈₀N₁₆O₁₆S | 1217,5736 | 609(2H+) |

| | | | | |
|---|---|---|---|---|
| Nomenklatur für Aminosäuren nach Eur.J.Biochem. 138, 9-37 (1984) ^{D}: HPLC-System: Lösungsmittel: A = 0,1 % TFA ; B = 80 % Acetonitril/ 0,1 % TFA; Gradient: 1 % B zu 99 % B in 50 min; Säule: Merck, LiChrosphere 60 250-4, RP-select B: 5 µm; Flussrate: 1 ml/min; Detektion: 215 nm. | | | | |

### Beispiel 4:

### Einfluß von löslichen Substanzen auf die Bindung von biotinyliertem Fibronektin an αᵥβ₆

Die hergestellten erfindungsgemäßen Peptide wurden in Lösung zusammen mit kompetetiv wirkenden Fibronektin an den immobilisierten αᵥβ₆ Rezeptor gebunden und der Q-Wert als Maß für die Selektivität der Bindung des zu testenden Peptids an αᵥβ₆ ermittelt. Der Q-Wert berechnet sich dabei aus dem Quotienten der IC₅₀-Werte von Testpeptid und einem Standard. Als Standard diente das lineare Ac-RTDLDSLR-NH₂ (WO01/00660; Pytela et al., 1986, Science 231, 1559). Der Bindungstest wurde im einzelnen wie folgt durchgeführt:

Die Immobilisierung von αᵥβ₆ auf Microtiterplatten erfolgte durch Verdünnung der Proteinlösurig in TBS++ und anschließender Inkubation über Nacht bei 4 °C (100 µl/Vertiefung). Unspezifische Bindungsstellen wurden durch Inkubation (2 h; 37 °C) mit 3 % (w/v) BSA in TBS++ (200 µl/Vertiefung) blockiert. Überschüssiges BSA wurde durch dreimaliges Waschen mit TBSA++ entfernt. Peptide wurden seriell (1:10) in TBSA++ verdünnt und zusammen mit biotinyliertem Fibronektin (2 µg/ml) mit dem immobilisierten Integrin inkubiert (50 µl Peptid + 50 µl Ligand pro Vertiefung; 2h; 37 °C).
Nicht gebundenes Fibronektin und Peptide wurden durch dreimaliges Waschen mit TBSA++ entfernt. Die Detektion des gebundenen Fibronektins erfolgte durch Inkubation (1 h; 37 °C) mit einem alkalische-Phosphatase-gekoppelten anti-Biotin Antikörper (1:20000 in TBSA++; 100 µl/Vertiefung). Nach dreimaligem Waschen mit TBSA++ erfolgte die kolorimetrische Detektion durch Inkubation (10-15 min; 25 °C; im Dunkeln) mit Substratlösung (5 mg Nitrophenylphosphat, 1 ml Ethanolamin, 4 ml H₂O (100 µl/Vertiefung)). Die Enzymreaktion wurde durch Zugabe von 0,4 M NaOH (100 µl/Vertiefung) gestoppt. Die Farbintensität wurde bei 405 nm im ELISA-Meßgerät bestimmt und gegen einen Nullwert abgeglichen. Als Nullwert dienten Vertiefungen, die nicht mit Rezeptor beschichtet waren. Als Standard wurde in jedem Versuch Ac-RTDLDSLR-NH2 eingesetzt, die einen IC₅₀-Wert < 100 nM besitzt.
IC₅₀-Werte für die getesteten Peptide wurden aus einer Graphik abgelesen und daraus zusammen mit der IC₅₀ des Standards der Q-Wert des Peptids ermittelt.
Bei diesen Versuchen zeigte sich, dass eine Vielzahl der synthetisierten Verbindungen submikromolare bis nanomlare Affinität zum Integrin αᵥβ₆ besitzt (siehe Tab. 1).

**Tab.1**

| **Sequenz** | **Q** |
|---|---|
| Ac-R-Dap(Psa)-DLDSLR-NH2 | 0,20 |
| Ac-R-Dap(F5-Psa)-DLDSLR-NH2 | 3,7 |
| Ac-R-Dap(2-NO2-Psa)-DLDSLR-NH2 | 0,27 |
| Ac-R-Dap(4-NO2-Psa)-DLDSLR-NH2 | 1,5 |
| Ac-R-Dap(2,4-NO2-Psa)-DLDSLR-NH2 | 1,5 |
| Ac-R-Dap(6-OMe-Psa)-DLDSLR-NH2 | 1,4 |
| Ac-R-Dap(2-CF3-Psa)-DLDSLR-NH2 | 0,29 |
| Ac-R-Dap(3-CF3-Psa)-DLDSLR-NH2 | 0,97 |
| Ac-R-Dap(Me5-Psa)-DLDSLR-NH2 | 0,97 |
| Ac-R-Dap(4-tBu-Psa)-DLDSLR-NH2 | 69 |
| Ac-R-Dap(Bsa)-DLDSLR-NH2 | 4,1 |
| Ac-R-Dap(1-Nap)-DLOSLR-NH2 | 0,71 |
| Ac-R-Dap(2-Nap)-DLDSLR-NH2 | 5,1 |
| Ac-R-Dap(4-Ph-Psa)-DLDSLR-NH2 | 4,8 |
| Ac-RTDLDSLR-NH2 | 1.00 |

| | |
|---|---|
| Q-Wert = IC₅₀ Testpeptid/IC₅₀ Standard Q-Werte aus Versuchswiederholungen wurden gemittelt | |

Die nachfolgenden Beispiele betreffen pharmazeutische Zubereitungen:

### Beispiel A: Injektionsgläser

Eine Lösung von 100 g der erfindungsgemässen Verbindung nach Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g erfindungsgemässen Verbindung nach Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g erfindungsgemässen Verbindung nach Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg erfindungsgemässen Verbindung nach Formel I mit 99,5 g Vaseline unter aseptischen Bedingungen.

### Beispiel E: Tabletten

Ein Gemisch von 1 kg erfindungsgemässen Verbindung nach Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg erfindungsgemässen Verbindung nach Formel I werden in üblicher Weise in Hartgelatinekapseln gefüllt, so daß jede Kapsel 20 mg des Wirkstoffs enthält.

### Beispiel H: Ampullen

Eine Lösung von 1 kg erfindungsgemässen Verbindung nach Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g erfindungsgemässen Verbindung nach Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Peptidische Verbindungen der Formel I
R¹-Arg-X-Asp-Leu-Asp-Ser-Leu-Arg-R² I
worin
R¹ H, Acetyl oder Acyl und
R² -OH, OR³ NH₂,NHR³, N(R³)₂
R³ Alkyl, Aralkyl, Aryl, Het und
X eine Aminosäure der Formel II
bedeutet, 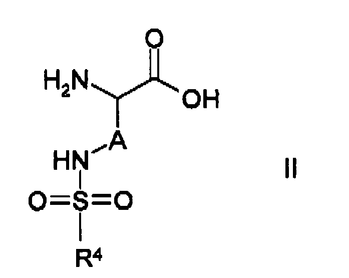 worin
A (CH₂)ₙ
R⁴ Aralkyl oder Aryl, und
n 1, 2, 3, 4, 5 oder 6
bedeutet,
und die Aminosäure der Formel II über eine Peptidbindung der α-Aminogruppe mit dem benachbarten Arg und über eine Peptidbindung der α-Carboxygruppe mit der α-Aminogruppe des benachbarten Asp verbunden ist, sowie deren pharmazeutisch verwendbare
a) Prodrugs, ausgewählt unter mit Alkyl- oder Acylgruppen, Zuckern oder Oligopeptiden abgewandelten Verbindungen der Formel I,
b) Derivate, ausgewählt unter den N-Methyl-, N-Ethyl-, N-Propyl-, N-Benzyl- und C_{α}-Methylderivaten sowie unter Derivaten, die N-terminal eine Acetylgruppe tragen oder in denen die Acetylgruppe durch eine andere Acylfunktion ersetzt ist, die ausgewählt ist unter Propionyl, Butyryl und Benzoyl,
c) Solvate,
d) Stereoisomere und
e) Salze
davon, wobei deren Mischungen in allen Verhältnissen umfasst sind.

2. Peptidische Verbindungen nach Anspruch 1 ausgewählt aus der Gruppe
Ac-Arg-Dap(Psa)-Asp-Leu-Asp-Ser-Leu-Arg-NH₂,
Ac-Arg-Dap(F5-PSA)-Asp-Leu-Asp-Ser-Leu-Arg-NH₂,
Ac-Arg-Dap(2-NO₂-PSA)-Asp-Leu-Asp-Ser-Leu-Arg-NH₂,
Ac-Arg- Dap(4-NO₂-PSA)-Asp-Leu-Asp-Ser-Leu-Arg-NH₂,
Ac-Arg- Dap(2,4-NO₂-PSA)-Asp-Leu-Asp-Ser-Leu-Arg-NH₂,
Ao-Arg-Dap(6-OMe-PSA)-Asp-Leu-Asp-Ser-Leu-Arg-NH₂,
Ac-Arg-Dap(2-CF₃-PSA)-Asp-Leu-Asp-Ser-Leu-Arg-NH₂,
Ac-Arg-Dap(3-CF₃-PSA)-Asp-Leu-Asp-Ser-Leu-Arg-NH₂,
Ac-Arg-Dap(Me₅-PSA)-Asp-Leu-Asp-Ser-Leu-Arg-NH₂,
Ac-Arg-Dap(4-tBu-PSA)-Asp-Leu-Asp-Ser-Leu-Arg-NH₂,
Ac-Arg-Dap(BSA)-Asp-Leu-Asp-Ser-Leu-Arg-NH₂,
Ac-Arg-Dap(1-Nap)-Asp-Leu-Asp-Ser-Leu-Arg-NH₂,
Ac-Arg-Dap(2-Nap)-Asp-Leu-Asp-Ser-Leu-Arg-NH₂,
Ac-Arg-Dap(4-Ph-Psa)-Asp-Leu-Asp-Ser-Leu-Arg-NH₂,
sowie deren pharmazeutisch verwendbaren Prodrugs und Derivate wie in Anspruch 1 definiert, Solvate, Stereoisomere und Salze davon, einschließlich deren Mischungen in allen Verhältnissen.

3. Verbindung der Formel I nach einem der Ansprüche 1 oder 2 zur Verwendung als Arzneimittel.

4. Arzneimittel enthaltend eine wirksame Menge an einer Verbindung der Formel I nach einem der Ansprüche 1 oder 2 neben gegebenenfalls einem oder mehreren inerten Trägerstoffen, Hilfsstoffen und/oder Verdünnungsmitteln.

5. Arzneimittel nach Anspruch 4, **dadurch gekennzeichnet, dass** mindestens ein weiterer Arzneimittelwirkstoff enthalten ist.

6. Verfahren zur Herstellung eines Arzneimittels gemäß Anspruch 4 oder 5, **dadurch gekennzeichnet, daß** auf nichtchemischem Wege eine Verbindung der Formel I nach einem der Ansprüche 1 oder 2 und gegebenenfalls ein weiterer Arzneimittelwirkstoff in einen oder mehrere inerte Trägerstoffe und/oder Verdünnungsmittel eingearbeitet wird.

7. Verwendung der Verbindungen der Formel I nach einem der Ansprüche 1 oder 2 zur Herstellung eines Medikaments zur Prophylaxe und/oder Bekämpfung von Erkrankungen, ausgewählt aus der Gruppe umfassend Erkrankungen des Kreislaufs, Fibrosen, Mucoviszidose, Leberzirrhose, Tumorerkrankungen, osteolytische Krankheiten, Entzündungen, ophthalmologische Krankheiten, rheumatische Arthritis, Osteoarthritis, ulcerative Colitis, Morbus Crohn, Atherosklerose, Psoriasis, Restenose, Multiple Sklerose und akutem Nierenversagen.

8. Verwendung nach Anspruch 7, **dadurch gekennzeichnet, dass** die Erkrankungen des Kreislaufs ausgewählt sind aus der Gruppe umfassend Lungenembolie, Thrombose, Herzinfarkt, Arteriosklerose, Aneurysma dissecans, vorübergehende ischämische Anfälle, Apoplex und Angina pectoris.

9. Verwendung der Verbindungen der Formel I nach einem der Ansprüche 1 oder 2 zur Herstellung eines Medikaments zur Beeinflussung von Wundheilungsprozessen.

10. Arzneimittelkit, bestehend aus getrennten Packungen von
a) einer wirksamen Menge einer Verbindung der Formel I nach einemder Ansprüche 1 oder 2 und
b) einer wirksamen Menge eines weiteren Arzneimittelwirkstoffes.

## Claims

1. Peptidic compounds of the formula I
R¹-Arg-X-Asp-Leu-Asp-Ser-Leu-Arg-R² I
in which
R¹ denotes H, acetyl or acyl and
R² denotes -OH, OR³, NH₂, NHR³, N(R³)₂
R³ denotes alkyl, aralkyl, aryl, Het and
X denotes an amino acid of the formula II 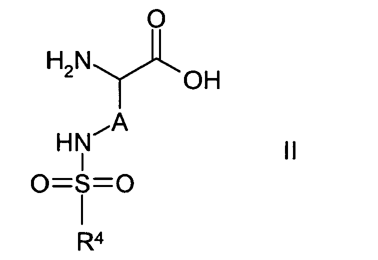
in which
A denotes (CH₂)ₙ
R⁴ denotes aralkyl or aryl, and
n denotes 1, 2, 3, 4, 5 or 6,
and the amino acid of the formula II is bonded to the adjacent Arg via a peptide bond of the α-amino group and to the α-amino group of the adjacent Asp via a peptide bond of the α-carboxyl group, and pharmaceutically usable
a) prodrugs selected from compounds of the formula I which have been modified by means of alkyl or acyl groups, sugars or oligopeptides,
b) derivatives selected from the N-methyl, N-ethyl, N-propyl, N-benzyl and C_{α}-methyl derivatives and from derivatives which carry an acetyl group in the N-terminal position or in which the acetyl group has been replaced by another acyl function selected from propionyl, butyryl and benzoyl,
c) solvates,
d) stereoisomers thereof and
e) salts thereof,
including mixtures thereof in all ratios are included.

2. Peptidic compounds according to Claim 1, selected from the group
Ac-Arg-Dap(Psa)-Asp-Leu-Asp-Ser-Leu-Arg-NH₂,
Ac-Arg-Dap(F5-PSA)-Asp-Leu-Asp-Ser-Leu-Arg-NH₂,
Ac-Arg-Dap(2-NO₂-PSA)-Asp-Leu-Asp-Ser-Leu-Arg-NH₂,
Ac-Arg- Dap(4-NO₂-PSA)-Asp-Leu-Asp-Ser-Leu-Arg-NH₂,
Ac-Arg- Dap(2,4-NO₂-PSA)-Asp-Leu-Asp-Ser-Leu-Arg-NH₂,
Ac-Arg-Dap(6-OMe-PSA)-Asp-Leu-Asp-Ser-Leu-Arg-NH₂,
Ac-Arg-Dap(2-CF₃-PSA)-Asp-Leu-Asp-Ser-Leu-Arg-NH₂,
Ac-Arg-Dap(3-CF₃-PSA)-Asp-Leu-Asp-Ser-Leu-Arg-NH₂,
Ac-Arg-Dap(Me₅-PSA)-Asp-Leu-Asp-Ser-Leu-Arg-NH₂,
Ac-Arg-Dap(4-tBu-PSA)-Asp-Leu-Asp-Ser-Leu-Arg-NH₂,
Ac-Arg-Dap(BSA)-Asp-Leu-Asp-Ser-Leu-Arg-NH₂,
Ac-Arg-Dap(1-Nap)-Asp-Leu-Asp-Ser-Leu-Arg-NH₂,
Ac-Arg-Dap(2-Nap)-Asp-Leu-Asp-Ser-Leu-Arg-NH₂,
Ac-Arg-Dap(4-Ph-Psa)-Asp-Leu-Asp-Ser-Leu-Arg-NH₂,
and their pharmaceutically usable prodrugs and derivatives as defined in Claim 1, solvates, stereoisomers thereof and salts thereof, including mixtures thereof in all ratios.

3. Compound of the formula I according to one of Claims 1 or 2 for use as medicament.

4. Medicament comprising an effective amount of a compound of the formula I according to one of Claims 1 or 2 besides optionally one or more inert excipients, adjuvants and/or diluents.

5. Medicament according to Claim 4, **characterised in that** at least one further medicament active compound is present.

6. Process for the preparation of a medicament according to Claim 4 or 5, **characterised in that** a compound of the formula I according to one of Claims 1 or 2 and optionally a further medicament active compound is incorporated into one or more inert excipients and/or diluents by non-chemical methods.

7. Use of the compounds of the formula I according to one of Claims 1 or 2 for the preparation of a medicament for the prophylaxis and/or combating of diseases selected from the group comprising circulatory diseases, fibroses, cystic fibrosis, cirrhosis of the liver, tumour diseases, osteolytic diseases, inflammations, ophthalmological diseases, rheumatic arthritis, osteoarthritis, ulcerative colitis, Crohn's disease, atherosclerosis, psoriasis, restenosis, multiple sclerosis and acute renal failure.

8. Use according to Claim 7, **characterised in that** the circulatory diseases are selected from the group comprising pulmonary embolism, thrombosis, cardiac infarction, arteriosclerosis, aneurysma dissecans, transient ischaemic attacks, apoplexy and angina pectoris.

9. Use of the compounds of the formula I according to one of Claims 1 or 2 for the preparation of a medicament for influencing wound-healing processes.

10. Medicament kit consisting of separate packs of
a) an effective amount of a compound of the formula I according to one of Claims 1 or 2 and
b) an effective amount of a further medicament active compound.

## Revendications

1. Composés peptidiques de formule 1
R¹-Arg-X-Asp-Leu-Asp-Ser-Leu-Arg-R² I
dans laquelle
R¹ désigne H, acétyle ou acyle et
R² désigne -OH, OR³, NH₂, NHR³, N(R³)₂
R³ désigne alkyle, aralkyle, aryle, Hét et
X désigne un acide aminé de formule II 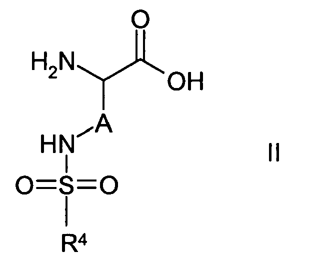
dans laquelle
A désigne (CH₂)ₙ
R⁴ désigne aralkyle ou aryle, et
n vaut 1, 2, 3, 4, 5 ou 6,
et l'acide aminé de formule Il est lié à l'Arg adjacent par l'intermédiaire d'une liaison peptidique du groupement α-aminé et au groupement α-aminé de l'Asp adjacent par l'intermédiaire d'une liaison peptidique du groupement α-carboxylé, et
a) les pro-drogues choisies parmi les composés de formule I ayant été modifiées au moyen de groupements alkyle ou acyle, de sucres ou d'oligopeptides,
b) les dérivés choisis parmi les dérivés N-méthylés, N-éthylés, N-propylés, N-benzylés et C_{α}-méthylés et parmi les dérivés portant un groupement acétyle en position N-terminale ou dans lesquels le groupement acétyle a été remplacé par une autre fonction acyle choisie parmi propionyle, butyryle et benzoyle,
c) les solvats,
d) les stéréoisomères de ceux-ci et
e) les sels de ceux-ci,
pharmaceutiquement utilisables, où des mélanges de ceux-ci selon des rapports quelconques sont inclus.

2. Composés peptidiques selon la revendication 1, choisis parmi le groupe
Ac-Arg-Dap(Psa)-Asp-Leu-Asp-Ser-Leu-Arg-NH₂,
Ac-Arg-Dap(F5-PSA)-Asp-Leu-Asp-Ser-Leu-Arg-NH₂,
Ac-Arg-Dap(2-NO₂-PSA)-Asp-Leu-Asp-Ser-Leu-Arg-NH₂,
Ac-Arg- Dap(4-NO₂-PSA)-Asp-Leu-Asp-Ser-Leu-Arg-NH₂,
Ac-Arg- Dap(2,4-NO₂-PSA)-Asp-Leu-Asp-Ser-Leu-Arg-NH₂,
Ac-Arg-Dap(6-OMe-PSA)-Asp-Leu-Asp-Ser-Leu-Arg-NH₂,
Ac-Arg-Dap(2-CF₃-PSA)-Asp-Leu-Asp-Ser-Leu-Arg-NH₂,
Ac-Arg-Dap(3-CF₃-PSA)-Asp-Leu-Asp-Ser-Leu-Arg-NH₂,
Ac-Arg-Dap(Me₅-PSA)-Asp-Leu-Asp-Ser-Leu-Arg-NH₂,
Ac-Arg-Dap(4-tBu-PSA)-Asp-Leu-Asp-Ser-Leu-Arg-NH₂,
Ac-Arg-Dap(BSA)-Asp-Leu-Asp-Ser-Leu-Arg-NH₂,
Ac-Arg-Dap(1-Nap)-Asp-Leu-Asp-Ser-Leu-Arg-NH₂,
Ac-Arg-Dap(2-Nap)-Asp-Leu-Asp-Ser-Leu-Arg-NH₂,
Ac-Arg-Dap(4-Ph-Psa)-Asp-Leu-Asp-Ser-Leu-Arg-NH₂,
et leurs pro-drogues et dérivés pharmaceutiquement utilisables tels que définis selon la revendication 1, les solvats, les stéréoisomères de ceux-ci et les sels de ceux-ci, y compris des mélanges de ceux-ci selon des rapports quelconques.

3. Composé de formule I selon l'une des revendications 1 ou 2 pour une utilisation comme médicament.

4. Médicament comprenant une quantité efficace d'un composé de formule I selon l'une des revendications 1 ou 2, outre éventuellement un ou plusieurs excipients, adjuvants et/ou diluants inertes.

5. Médicament selon la revendication 4, **caractérisé en ce qu'**au moins un autre composé actif médicamenteux est présent.

6. Procédé de préparation d'un médicament selon la revendication 4 ou 5, **caractérisé en ce qu'**un composé de formule I selon l'une des revendications 1 ou 2 et éventuellement un autre composé actif médicamenteux est incorporé dans un ou plusieurs excipients et/ou diluants inertes par des méthodes non chimiques.

7. Utilisation des composés de formule I selon l'une des revendications 1 ou 2 pour la préparation d'un médicament destiné à la prophylaxie et/ou la lutte contre des maladies choisies parmi le groupe constitué par les maladies circulatoires, les fibroses, la mucoviscidose, la cirrhose du foie, les maladies tumorales, les maladies ostéolytiques, les inflammations, les maladies ophtalmologiques, la polyarthrite rhumatoïde, l'arthrose, la rectocolite hémorragique, la maladie de Crohn, l'athérosclérose, le psoriasis, la resténose, la sclérose en plaques et l'insuffisance rénale aiguë.

8. Utilisation selon la revendication 7, **caractérisée en ce que** les maladies circulatoires sont choisies parmi le groupe constitué par l'embolisme pulmonaire, la thrombose, l'infarctus du myocarde, l'artériosclérose, l'anévrysme disséquant, les accidents ischémiques transitoires, l'apoplexie et l'angine de poitrine.

9. Utilisation des composés de formule I selon l'une des revendications 1 ou 2 pour la préparation d'un médicament destiné à influencer les processus de guérison de plaies.

10. Kit médicamenteux constitué de conditionnements séparés
a) d'une quantité efficace d'un composé de formule I selon l'une des revendications 1 ou 2 et
b) d'une quantité efficace d'un autre composé actif médicamenteux.
